# EUROPEAN PATENT APPLICATION

(11) **EP 0 691 136 A2**
(43) Date of publication of application: **10.01.1996**
(21) Application number: 95201634.3
(22) Date of filing: 11.05.1993
(51) Int. Cl.: A61M 16/04

(54) **Tracheotomy cannula**

(30) Priority: 11.05.1992 DK 610/92
(71) Applicant: JEPPESEN, Finn, DK-8800 Viborg (DK)
(72) Inventor: JEPPESEN, Finn, DK-8800 Viborg (DK)
(74) Representative: Christiansen, Ejvind

(57) **Abstract**

A tracheotomy cannula comprising an inner and an outer tube (1, 11), a ring element (20, 30, 40) as a holding device, and a speech valve (50).

The ring element (20, 30, 40) is provided with slots (23, 33, 42) wherein the flange (3) of the outer tube is releasably engaged. The advantage of using a ring element rather than a plate is that the contact area between the ring element and the skin is considerably less, which improves the hygiene of using the tracheotomy cannula. Furthermore, the ring element is more slender than a plate, which means that the tracheotomy cannula is less conspicuous in use.

## Description

The present invention relates to a tracheotomy cannula comprising a holding device as well as an outer and an inner tube which are both equipped with a flange, and wherein the holding device can be fastened to the neck of a patient by means of a band or the like and is equipped with a central aperture around an insertional opening for insertion of the outer tube, which is kept in place at the neck of the patient by means of the holding device, and wherein the outer tube is equipped with locking devices for securing the inner tube, and wherein the inner tube may optionally be equipped with devices for holding a speech valve.

In order to prevent the outer tube from being displaced from the cervix and throat, e.g. during coughing or when the patient is asleep, it is necessary that the outher tube be secured to the neck,

U.S. Patent No. 3,088,466 and U.S. Patent No. 3,688,774 both disclose a tracheotomy cannula consisting of an outer and an inner tube which can be separated and wherein both tubes have a flange.

In U.S. Patent No, 3,088,466 the flange of the outer tube is provided with holes for attaching a band or the like for keeping the cannula in place on the neck. In U.S. Patent No. 3,688,774 both the inner and the outer tubes are inserted through a more narrow hole in a loose resilient plate which is also intended for the fastening of bands or the like. Both cannulas satisfy the patient's need for taking out the inner tube in order to clean it of mucous secretion etc. which will accumulate in the tube when used. However, the outer tube's flange consists in a plate which, due to its contact with the skin, collects humidity and impurities and therefore is badly suited from a hygienic point of view.

Furthermore, tracheotomy cannulas are known which also comprise an outer tube with a plate to which bands or the like may be fastened and which also comprise an inner tube which is separable from the outer tube, wherein, attached to the inner tube, is a speech valve consisting of a short tube in which a flap is mounted. This flap is hinged to the top of the tube and is able only to tip towards the trachea, whereby the valve functions as a one-way valve. Inhalation thus passes through the tracheotomy cannula whereas exhalation passes through the ordinary respiratory organs, whereby the patient is enabled to speak.

In many of the known tracheotomy cannulas, the outer tube is equipped with small tongues which engage with a brace on the carrier plate in such a way that the outer tube is able to wriggle slightly in relation to the plate. In some of these cannulas the outer tube and the carrier plate cannot be separated so that cleaning the cannula itself and the area around the hole in the plate is impeded and becomes ineffective. In other known cannulas the outer tube and the carrier plate may be separated, albeit with some difficulty.

U.S. Patent No. 2,039,142 discloses a tracheotomy cannula in which that part of the cannula which is on the outside of the neck is designed as a piece of jewellery. The outer tube of this cannula is designed similar to a brooch consisting of a casing with a cover which is designed as a very ingeniously perforated lid. The disadvantage of this cannula design is that the construction is big and therefore only seems natural when worn by women - most male persons would not like to wear such a big piece of jewellery. Besides, the brooch-like encapsulation means that the area which is in contact with the skin is excedingly big and somewhat bigger than the area of the actual flange of the outer tube.

The disadvantages of the known tracheotomy cannulas are that they are unhygienic in use because the flange of the outer tube, which is relatively big in order to secure that the cannula will not disappear into the throat, represents a source of accumulations of mucus, sweat, and other impurities between the flange and the neck. This may be the basis of an infection or some other disease, e.g. general irritation of the skin. In order to insert the cannula into the throat to the trachea, a permanent hole is made in the neck. An infection around this hole may risk to spread to the hole itself which is why it is essential that the area surrounding the hole be kept clean. The patient cannot by himself remove the outer tube, which means that it is only possible to do an effective cleaning behind the flange in connection with the patient's consulting a doctor. The patient may, however, use small bandages which absorb moisture but they emphasize the look of a medical aid.

Besides the big flange is unpleasant because of its dominating and technical appearance in the middle of the neck and since the cannula is worn by the patient every day, it is a great mental nuisance to the wearer. Therefore, the patient will often wear thin scarves to hide the cannula.

The object of the present invention is to disclose a tracheotomy cannula whose constructive design eliminates or to a considerable degree reduces the above-mentioned disadvantages in order to improve both the physical and the mental well-being of the patient. Physical well-being is improved by reducing the risk that impurities will accumulate around the outer tube and its flange. Mental well-being is improved by making the parts of the cannula placed on the outside of the neck as slender as possible and by giving the parts a decorative appearance so that it will not be visible to the same extent that the patient is suffering from a handicap. The flange of the outer tube must, however, be big enough to cover the actual hole in the neck.

This object is fulfilled by the present invention which is characterized in that the fastening device is ring-shaped and plane or planocurved and is equipped with slots which may, by mutual turns of the fastening device and the outer tube flange mainly in level with the flange, be releasably engaged with the rim of the flange.

The feature of the present invention is that the ring element is slender and thus not so dominating as a flange consisting of a plate with the same dimensions. Besides the area in contact with the neck is reduced when, as in the present invention, a ring element is used rather than a plate and the hygienic conditions are thus considerably improved.

The above features make the tracheotomy cannula according to the present invention more user-friendly both mentally and physically. The use of a ring element for holding the cannula in place in the neck leads to a more slender design and consequently less dominance in the neck. This means that the wearer will not feel as much mental discomfort from wearing the cannula. The ring element and the actual cannula may advantageously be designed in such a manner that the cannula appears as a piece of jewellery. Therefore the cannula is primarily produced in silver, gilted silver, stainless steel, or some material which may be anodized in optional colours, e.g. titanium. Using stainless steel and particularly titanium, the weight of the cannula may be reduced because the dimensions of the materials may be reduced for the ring element as well the flanges and the tubular parts of the outer and inner tubes. Other materials than metallic ones may also be used for the production of the cannula, e.g. plastics. In choosing materials it has also been taken into account that the material must not cause the wearer to have allergic reactions.

Besides the cannula according to the invention will, as mentioned, be more hygienic because the ring element is the only element to be in contact with the neck. The area of the cannula in contact with the neck is thus considerably smaller than in known cannulas. The contact area of the cannula according to the invention will at the same time be further apart from the hole leading into the trachea through the neck. The flange of the outer tube which engages the slots in the ring element in a marginal rim area will not be in close contact with the neck when the slots are designed as almost radially situated slots which cover part of the periphery of the ring element. Thus the construction will make it possible to create some distance between the neck and the back of the flange of the outer tube. This helps to reduce the risk that impurities will accumulate around the cannula. This will be seen from the description below of a design according to the invention.

The slots formed in the ring element are at least in part of their extent designed with slightly more width than the thickness of the flange of the outer tube. When the flange of the outer tube is engaging with the slots, it is thus made possible that the outer tube may perform small sideways rocking or swaying movements. This improves the comfort of wearing the tracheotomy cannula according to the invention because the rocking movement allows the wearer for example to turn his head slightly without causing tensions to occur around the cannula and the neck due to an excessively rigid securing of the cannula.

The "loose" engagement of the ring element slots and the flange of the outer tube also means that it will be easier to separate the two parts, which is necessary when cleaning the area around the hole in which the cannula is inserted as well as when individual parts are cleaned and polished.

In order to further improve the comfort of wearing the tracheotomy cannula according to the invention, the ring element is equipped with more or less curvedness around a vertical axis so that the ring element is in perfect contact with that part of the neck's circumference which the ring element covers. If the tracheotomy cannula according to the invention is used on a person with a solid neck, It is almost unnecessary to give the ring element any curve because the curvedness of that person's neck will be zero or next to zero over the part of the neck covered by the ring element. If, on the other hand, the tracheotomy cannula is worn by a person with a slender neck, the curvedness of the neck over the area covered by the ring element will have a certain magnitude which is why the ring element must be given a corresponding curve so that the ring element will have contact over the whole of its prospective contact area.

The above-mentioned characteristics of the tracheotomy cannula according to the invention make sure that a cannula is produced which has a higher degree of user friendliness than previously known cannulas. As mentioned, the cannula according to the invention is both more hygienic and more pleasant and consequently also more suited as an everyday tool for living with a handicap.

### DESCRIPTION OF THE DRAWING

The invention will now be further explained with reference to the accompanying drawing, wherein
- fig. 1: is a perspective view of an outer tube for use in connection with the tracheotomy cannula according to the invention,
- fig. 2: is a perspective view of a first embodiment of an inner tube for use in connection with the tracheotomy cannula according to the invention,
- fig. 2A: is a perspective view of a second embodiment of an inner tube for use in connection with the tracheotomy cannula according to the invention,
- fig. 3: is a perspective view of a first embodiment of the ring element for use in connection with the tracheotomy cannula according to the invention,
- fig. 4: is a perspective view of an alternative embodiment of a ring element,
- fig. 5: is a perspective view of a third embodiment of a ring element,
- figs. 6A, 6B, 6C and 6D: are schematic plane views of ring elements according to the invention, situated on the wearer's neck,
- figs. 7A and 7B: are plane views of a speech valve intended for mounting on the inner tube, and
- fig. 8: is a perspective view showing how a ring element and an outer tube according to the invention will interact.

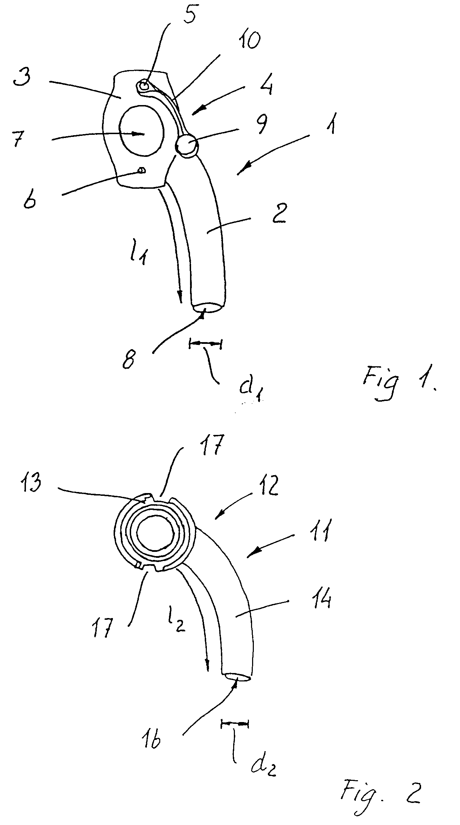

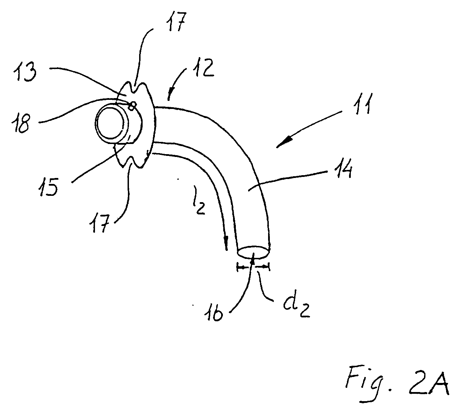

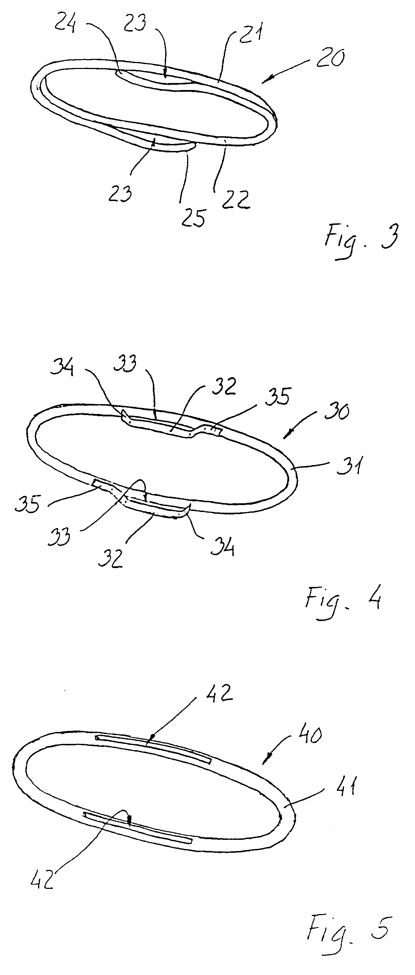

Fig. 1 illustrates an outer tube 1 which forms part of the tracheotomy cannula according to the invention. The outer tube comprises a tubular part 2 and a flange 3. In addition, on the flange 3 of the outer tube 1, a bolt 4 is mounted which turns around an axis 5 and which is intended for holding the flange of an inner tube (see fig. 2). Furthermore, mounted on the flange 3 of the outer tube, there is a stop pin 6 whose function will be explained below. The flange of the outer tube is almost elliptic with a minor axis a₁ and a major axis b₁. The elliptic shape is the preferred shape but other shapes may also be used. The shape of the flange 3 of the inner tube depends primarily on the shape of a ring element (see figs. 3A and 3B) which the flange engages.

The tubular part 2 of the outer tube is curved in order to facilitate its insertion into a hole in the neck of the patient who is to wear the tracheotomy cannula. The cross section of the tube 2 around an external opening 7 is almost perpendicular to the cross section of the tube 2 on an internal opening 8 which is situated inside the trachea. The length l₁ and diameter d₁ of the tubular part 2 vary depending on the person who is to wear the tracheotomy cannula - i.e. primarily whether it is a child or an adult person.

The flange 3 is fastened perpendicularly to the external opening 7 of the tubular part 2. The uttermost end of the bolt 4 which is fastened to the flange 3 is equipped with a jewel 9 which gives this part of the tracheotomy cannula a pleasant appearance. Alternatively, the bolt 4 may be equipped with a silver botton or the like which is able to fulfil the same object as the jewel. Along part of its extent the bolt 4 is shaped as a curve 10 so that when the bolt is moved over the flange of the inner tube, this curve 10 will be situated along another tubular part of the inner tube (see fig. 2). The stop pin 6 consists in a stick or a pin which is protruding from the flange 3 of the outer tube.

The outer tube 1 will primarily be made from or covered with a material which will not cause the wearer to have allergic reactions. Thus it will mostly be silver or gold or other metals, e.g. titanium, which at the same time give the cannula a decorative appearance. If titanium is used, it will be possible to reduce the thickness of the cannula materials and consequently their weight.

Fig. 2 illustrates an inner tube 11, similarly consisting of a tubular part 12 and a flange 13. The tubular part 12 consists of one tubular part 14 which has a curve equal to the tubular part 2 of the outer tube 1, and which is intended for insertion into the outer tube. To insert the tube, the end 16 of the inner tube's curved tubular part 14 which is most distant from the inner tube's flange 13 is inserted into the end 7 of the tubular part 2 of the outer tube 1 on which the flange 3 of the outer tube is mounted. The inner tube 11 is wholly inserted until the back of the inner tube's flange 13 is in contact with the front of the outer tube's flange 3. Thereafter, the bolt 4 mounted on the flange 3 of the outer tube 1 is pressed over the flange 13 of the inner tube in order to retain the inner tube 11 in the outer tube 1. The length l₂ of the inner tubular part 14 of the inner tube, measured from the flange 13 down the curved part, is shorter than the length l₁ of the tubular part 2 of the inner tube 1. Thus the inner tube 11 will not extend beyond the utmost end 8 of the outer tube 1, at the opposite end of its flange 3. The diameter d₂ of the tubular part 14 of the inner tube is slightly smaller than the diameter d₁ of the tubular part 2 of the outer tube 1 so that the inner tube 11 is insertable into the outer tube 1.

The flange 13 of the inner tube is, like that of the outer tube, almost elliptic, but this flange, too, may have other shapes. The flange 13 is equipped with incisions 17 in order to make room for the stop pin 6 and the axis 5 of the bolt 4, which are both mounted on the flange 3 of the outer tube 1, when the inner tube is inserted into the outer tube. The flange 13 of the inner tube is mainly to prevent the inner tube from sliding into the tubular part 2 of the outer tube 1. Besides, the flange 13 of the inner tube serves to fasten the inner tube to the outer tube as the flange is a contact face is the bolt 4. Thus it is not possible for the inner tube to move out of the outer tube when the bolt 4 is placed before the flange 13 of the inner tube.

The second tubular part 15 of the inner tube, extending from the other side of the flange 13, is shorter than the curved tubular part 14. This second tubular part 15 is intended for fastening a speech valve, if any, with adequate fastening devices, e.g. a thread, a bayonet socket, or simply a press fit between the other tubular part 15 and a corresponding tubular part of a speech valve.

Fig. 2A discloses an embodiment illustrating the male part of a bayonet socket consisting of two diametrically opposed pins 18 (only one shown) intended to engage with slots in a tubular part of a speech valve (see fig. 4). The pins are situated on another tubular part 15 extending from the other side of the flange 13.

Fig. 3 illustrates a ring element 20 for use in connection with the outer and inner tubes, 1 and 11, described above. The ring element 20 is made up of two parts 21, 22 of separate rings, united by welding, soldering, gluing or some other method, and the ring element 20 is elliptic in the illustrated embodiment. The ring element in the illustrated embodiment is plane but, as mentioned above, it may be curved around its minor axis in order to adapt to the curve of the neck surface. The two tubular parts 21, 22 both cover more than half of the periphery of the ring element 20 so that in parts of the periphery of the ring element they overlap. Along the peripheral part of the ring element 20 where the two separate ring parts 21, 22 overlap, gaps appear between the two parts and form slots 23 in which the flange 3 of the outer tube 1 is engaging (see fig. 1). In order to enable the outer tube 1 to perform sidewards rocking movements when its flange 3 is engaged in the slots 23, the two separate united ring elements 21, 22 are bended along the part of their extent where they overlap. The bend is made in a way that will form a permanent slot which has a width slightly larger than the thickness of the flange 3 of the outer tube 1 and in such a way that at the slot openings a stop is formed by the extreme 24, 25 of the separate ring part 21, 22 being in contact with the other separate ring part 22, 21, which it overlaps.

The ring element 20 is produced from a springy material since either of the separate ring parts 21, 22 which is bended and whose extreme 24, 25 is in contact with the other separate ring part must be able to open for introduction of the flange 3 of the outer tube into the slots 23 and then to close again in order to retain the flange of the outer tube in the slots. Besides the material must fulfil certain secondary requirements, e.g. the material must not provoke allergic reactions on contact with the wearer's skin.

Fig. 4 illustrates an alternative design of a ring element. The ring element 30, likewise elliptic and plane, consists of a closed ring 31 equipped with resilient braces 32. The braces are fastened to the same axial side but on opposite peripheral sides of the ring element. The braces 32 are bended so that they fulfil the same object as the bending of the separate ring parts 21, 22 in the embodiment in figure 3. Slots 33 are formed to receive the flange 3 of the outer tube 1 (see fig. 1) as well as a stop 34 which ensures that the flange will not unintendedly get out of its engagement with the ring element 30. The ring element 30 and the springy braces 32 are joined along the feet 35 of the braces by welding, soldering, gluing, or some other method.

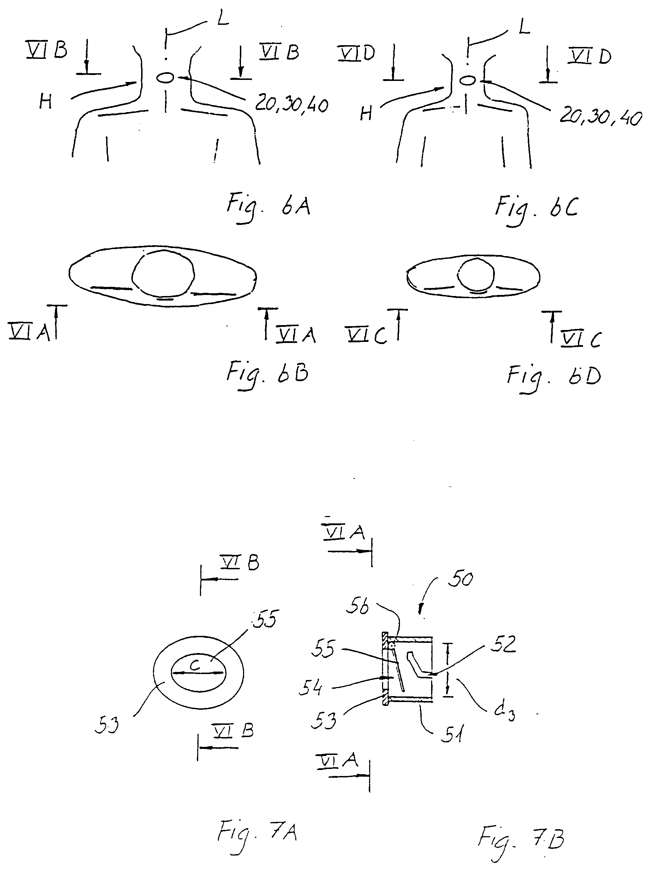

Fig. 5 illustrates a third and preferred embodiment of a ring element. The ring element 40 in this embodiment consists of a closed ring 41 whose slots 42 consist of milled holes longitudinally along the plane of the ring element. The ring element 40 in this embodiment is elliptic and plane as well, but other shapes may also be used. The slots 42 are formed along the major axis of the ellipse and on either side of the minor axis so that the ring element is symmetrical around both the major and the minor axis. However, it will be possible to situate the slots 42 in other places along the periphery of the ring element. The slots 42 are wider than the thickness of the flange 3 of the outer tube 1 in order to enable the outer tube to perform rocking movements.

Like the ring elements 20, 30 of the preceding figures, the ring element 40 illustrated in this figure is produced from a material which will not cause the wearer to have allergic reactions. No further requirements are made of the material or the dimensions as long as parametres such as strength, wear-resistance and other physical features are taken account of. Considering the desirability of the ring element being as slender as possible, the element will primarily be produced from a material with great strength, such as stainless steel or titanium, in order to make it possible to keep the dimensions as small as possible.

Figs. 6A-6D illustrate schematically how the ring element 20, 30, 40 according to the invention may be curved around an axis A parallel with the longitudinal axis L of the neck H of the person wearing the tracheotomy cannula. If the ring element is elliptic, as shown in the figures, the ring element is curved around the minor axis.

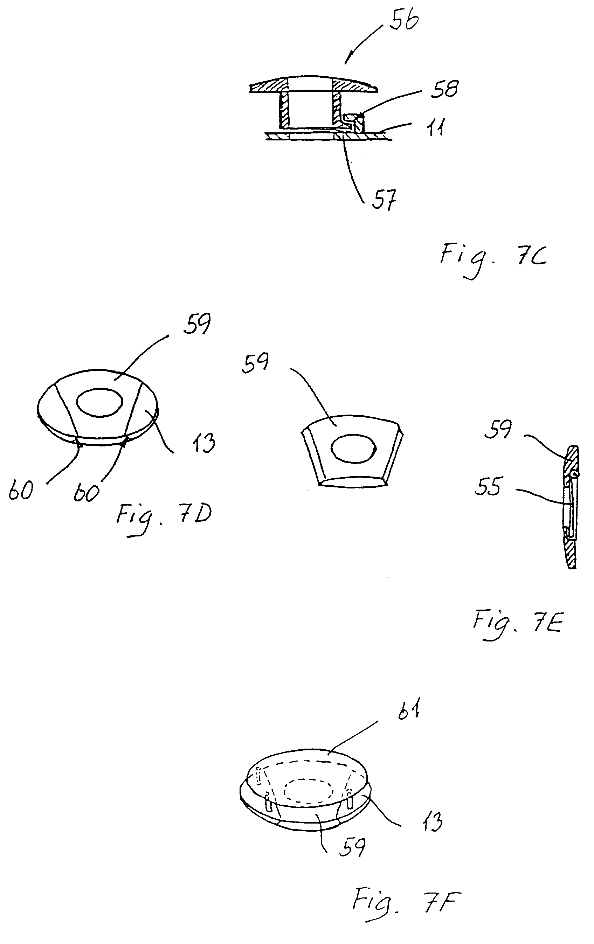

Figures 7A and 7B illustrate a speech valve 50 for use in connection with the inner tube 11, thus forming part of the tracheotomy cannula according to the invention. The speech valve 50 consists of a tubular part 51 with an inner diameter d₃ approximately as big as the outside diameter of the second tubular part 15 of the inner tube 11. In the tubular part 51 of the speech valve 50 tracks 52 are made which form the female part of a bayonet socket and which are intended to engage with the pins 18 of the second tubular part 15 of the inner tube 11 which form the male part of the bayonet socket. At one end of the tubular part 51 of the speech valve 50 a cover plate 53 with an oval hole 54 is placed. On the inside of this cover plate 53 an oval flap 55 is mounted, hinged in the uppermost part of the cover plate and with an axis of rotation 56 of the hinge parallel with the maximum distance c between two opposite points on the periphery of the oval flap 55. The flap 55 is oval in horizontal direction, i.e. the maximum distance c is in horizontal direction. Along its entire extent, the oval flap 55 overlaps the periphery of the oval hole 54. This way the flap 55 is only able to turn one way, viz. inwards and away from the the cover plate 53 of the speech valve 50.

The advantage of the illustrated speech valve 50 is that it will offer less resistance than a valve with a circular flap and with the same cross-sectional area as the oval flap 55 because the inertia force of the oval flap is less than that of a circular flap.

Fig. 7C illustrates an alternative way of fastening a speech valve 56 on the inner tube 11. The fastening is made by equipping the speech valve 56, which in the illustrated embodiment consists of a tubular valve, with a flap 57 which is intended to engage with a groove 58 formed on the outside of the flange 13 of the inner tube 11. The speech valve 56 is mounted and dismounted by rotating the valve so that the flap 56 will engage and disengage, respectively, with the groove 58.

Figures 7D and 7E illustrate an additional alternative way of fastening a speech valve 59 on the inner tube 11. The speech valve 59 in this embodiment consists of a slide intended to be pressed into a corresponding track 60 formed in the flange 13 of the inner tube 11. The slide has a dovetailed cross section and is conical in a direction parallel with the direction, illustrated by the arrow, in which the speech valve 59 is pressed into the track 60. This design also ensures that the speech valve will be kept in engagement with the inner tube while at the same time it is possible to dismount it for cleaning.

The above-mentioned two alternative valves are, like the valve illustrated in figs. 7A and 7B, equipped with a flap 55, e.g. as shown in fig. 7E.

In order to further increase the decorative design of the tracheotomy cannula, the speech valves may be equipped with a jewel 61 or some similar decoration which will visually cover the hole in the speech valve, like illustrated in fig. 7F. The jewel is mounted on the speech valve in such a manner that the possibility of breathing through the valve is maintained.

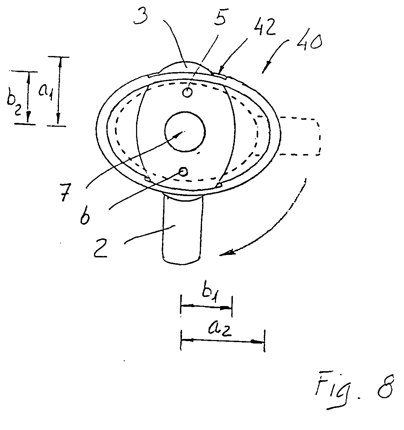

The following sections will primarily describe how the ring element interacts with the outer tube to form the tracheotomy cannula according to the invention. Reference is made to fig. 8.

A ring element 40 like the one described under fig. 5 consists of a closed elliptic ring with slots 42 along opposite parts of the periphery on either side of the minor axis b₂. This ring element has an aperture with an elliptic cross section of a given cross-sectional area. The flange 3 of the outer tube 1 has a cross section which is smaller than the cross section of the elliptic opening and which allows in at least one position that the flange 3 is inserted into the aperture of the ring element 40 while at the same time the plane of the flange is parallel with the plane of the ring element. In the shown embodiments of the outer tube 1 and the ring element 40 this will be in the cases when the major axis a₂ of the ring element coincides with the major axis a₁ of the flange 3 of the outer tube.

When the flange 3 of the outer tube has been inserted exactly so far into the aperture of the ring element 40 that the plane of the ring element largely coincides with the plane of the flange, the outer tube is rotated 90° around its axis, which is perpendicular to the flange. In this way the major axis a₁ of the flange 3 is made to coincide with the minor axis b₂ of the ring element 40. The major axis a₁ of the flange 3 is longer than the minor axis b₂ of the aperture of the ring element 40 and on the above-mentioned rotation the flange will thus be engaged with the slots 42 formed in the periphery of the ring element so that the outer tube 1 is secured to the ring element 40.

The axis 5 around which the bolt 4 (not shown) mounted on the flange 3 of the outer tube is turned ensures, together with the stop pin b, that the outer tube cannot be displaced forwards or backwards along the minor axis b₂ of the ring element, which would imply a risk that the flange might loosen from its engagement with the slots of the ring element 40.

Subsequently, the inner tube 11 (not shown) may be inserted into the outer tube 1 and the bolt 4 of the flange 3 of the outer tube is moved before the flange 13 of the inner tube in order to retain the inner tube in the outer tube. Then the speech valve 50 (not shown) may be mounted on the second tubular part 15 of the inner tube.

As appears from the above description of how the individual parts of the tracheotomy cannula interact, it is necessary for the flange of the outer cannula to be inserted into the opening of the ring element and subsequently to be rotated and brought into engagement with the slots of the ring element. This may be accomplished with other geometric shapes than the elliptic shape of the ring element and the flange of the outer tube, respectively. E.g. the above-mentioned mounting may also be performed with rectangular ring elements and flanges. Consequently, it is not to be taken as a limitation of the invention that the shown designs of these two parts are illustrated as being elliptic.

## Claims

1. A tracheotomy cannula comprising a holding device (20, 30, 40) as well as an outer (1) and an inner tube (11) which are both equipped with a flange (3,13), and wherein the holding device (20,30,40) can be fastened to the neck (H) of a patient by means of a band or the like and is equipped with a central aperture around an insertional opening for insertion of the outer tube (1), which is kept in place at the neck (H) of the patient by means of the holding device, and wherein the outer tube (1) is equipped with locking devices (14) for securing the inner tube (11), and wherein the inner tube may optionally be equipped with devices (15) for holding a speech valve (50), **characterized** in that the holding device (20,30,40) is ring-shaped and plane or planocurved and is equipped with slots (23,33,42) which are, by mutual turns of the holding device and the outer tube flange (3) mainly in level with the flange, releasably engaged with the rim of the flange.

2. A tracheotomy cannula according to claim 1, **characterized** in that the fastening device (40) consists of a closed ring (41) and that the slots (42) in the ring are formed by slotting part of the periphery of the ring in a plane parallel to the plane of the ring.

3. A tracheotomy cannula according to claim 1, **characterized** in that the fastening device (20) is composed of united parts (21, 22) which overlap each other in part of their extent and that the slots (23) are formed in the gap between the overlapping parts.

4. A tracheotomy cannula according to claim 1, **characterized** in that the holding device (30) consists of a closed ring (31) to one axial side of which springy braces (32) are attached, and that the slots (33) are formed in the gaps between the springy braces (32) and one side of the closed ring to which the braces are attached.

5. A tracheotomy cannula according to any of the preceding claims, **characterized** in that the flange (3) of the outer tube (1) has an outside cross-sectional area which is less than the inside cross-sectional area of the holding device (20, 30, 40), and that the outside periphery of the outer tube flange can be made to cut the in-
